# EUROPEAN PATENT APPLICATION

(11) **EP 1 770 094 A1**
(43) Date of publication of application: **04.04.2007**
(21) Application number: 05108855.7
(22) Date of filing: 26.09.2005
(51) Int. Cl.: C07D 493/10

(54) **Efficient synthesis of asymmetric epoxidation catalyst I**

(71) Applicant: Institut Catala D'Investigacio Quimica, 43007 Tarragona (ES); ICREA, Institució Catalana de Recerca I Estudis Avançats, Passeig Lluís Companys, 23 Barcelona CP 08010 (ES)
(72) Inventor: Vidal i Ferran, Anton Dr., Av. Paisos Catalans, 16 430007 Tarragona (ES); Nieto Alonso, Natàlia, Av. Paisos Catalans, 16 430777 Tarragona (ES); Molas Porqueras, Pineda Dr., Av. Paisos Catalans, 16 430007 Tarragona (ES)
(74) Representative: Barlocci, Anna

(57) **Abstract**

A process for the preparation of chiral ketone I from diol II comprising the acetylation of diol II with an acetylating agent, preferably an acetic acid derivative, wherein said acetylation is performed in the presence of a Lewis acid. The process can further comprise additional previous steps, so that chiral ketone I can be prepared in four steps starting from D-fructose. Alternatively, a process for the preparation of chiral ketone V from diol VI comprising the acetylation of diol VI with an acetylating agent, preferably an acetic acid derivative, wherein said acetylation is performed in the presence of a Lewis acid. The process can further comprise additional previous steps, so that chiral ketone V can be prepared starting from L-fructose, L-sorbose or D-arabinose.

## Description

### Field of the invention

The present invention is directed to a practical and scalable process for the preparation of chiral ketone I and its enantiomeric counterpart. Chiral ketone I is an efficient catalyst for the asymmetric epoxidation of alkenes.

### Background art

Optically active epoxides are highly versatile intermediates that can be converted into a wide variety of substantially enantiomerically pure molecules. Among the various methods that have been developed for the preparation of chiral epoxides, the asymmetric epoxidation of alkenes provides a powerful approach to the synthesis of said epoxides.

In recent years, chiral dioxiranes have been shown to be unrivaled agents for the asymmetric epoxidation of specific kinds of alkenes (see for example: Adam, W.; Curci, R.; Edwards, J. O. Acc. Chem. Res. 1989, 22, 205-211). Dioxiranes are usually generated from Oxone (potassium peroxomonosulfate) and ketones. The epoxidation can be performed using either isolated dioxiranes or dioxiranes formed *in situ* from catalytic amounts of a chiral ketone which is regenerated upon epoxidation (see for example: Jacobsen, E. N.; Wu, M. H. *Comprehensive Asymmetric Catalysis I-III*; 1^{st} ed)

Following the first asymmetric epoxidation using a chiral ketone reported by Curci (Curci, R.; Fiorentino, M.; Serio, M. R. Chem. Commun. 1984, 155-6), numerous chiral ketones have been studied and reported. Outstanding work in this area has been published by Shi's group, which has led to the development of highly efficient epoxidation catalysts with the general structure 1 (see the European patent EP 1 021 426 B1). Fructose-derived ketone **III,** which is commercialy available, is the most-well known asymmetric catalyst from this series, yielding high enantioselectivities in the epoxidation of a wide variety of *trans-, cis-* and trisubstituted alkenes (see for example: Shi, Y. Acc. Chem. Res., 2004, 37, 488-496).

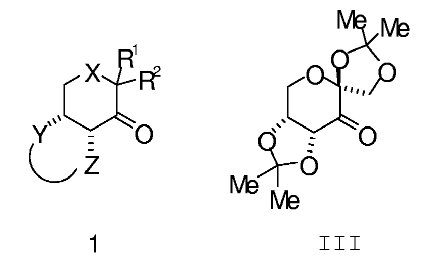

However, ketone III is not effective towards α,β-unsaturated esters, probably due to the low reactivity of electron-eficient alkenes towards epoxidation and to the decomposition of the catalyst under the reaction conditions. Taking into account the importance of chiral α,β-epoxy esters as key intermediates for the synthesis of complex molecules, it was necessary to find an effective catalyst for the asymmetric epoxidation of α,β-unsaturated esters, as well as other electron deficient alkenes. As used herein, the term "electron deficient alkene" refers to an alkene having at least one electron withdrawing group directly bonded to the olefinic moiety. By the term "withdrawing group" is intended "a group with a positive sigma value as defined by Hammett's Equation". Some examples of electron withdrawing groups comprise esters, ketones, sulfones, nitriles and nitro groups. As used herein, the term "non-electron deficient alkene" refers to an alkene having no electron withdrawing group directly bonded to the olefinic moiety.

Currently, only a few asymmetric epoxidation methods are available for epoxidazing electron deficient alkenes. Unfortunately, most methods described in the background art have shortcomings, such as the need to use transition metal catalysts, low selectivity or low yield. The only example of catalytic asymmetric epoxidation of electron deficient alkenes by using dioxiranes generated *in situ* has been recently described by Shi (see the international patent application WO 03/066614). Shi has found that chiral ketone **I** is active and highly enantioselective for the catalytic epoxidation of electron deficient alkenes, in particular for the epoxidation of α,β-unsaturated esters.

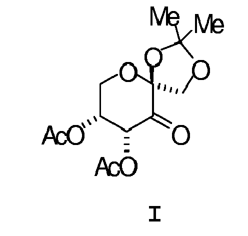

However, the preparation of chiral ketone **I** with high yield and purity still remains a drawback. Shi has reported the preparation of chiral ketone **I** through diacetylation of diol **II** with acetic anhydride and catalytic amounts of DMAP (see scheme 1). The reproduction of said preparation process at the inventor's laboratory showed that the elimination product **IX** was always formed in variable amounts together with the desired chiral ketone **I.** The amount of by-product **IX** formed depended on the amount of the catalyst: whereas the use of 2 mol% of the catalyst afforded a mixture of chiral ketone **I** and elimination product **IX** 64.4:35.6, the use of 20 mol% of DMAP afforded substantially the elimination product **IX** (see example)

On the other hand, the use of sodium acetate under microwave irradiation has been reported to be a useful catalyst for the acetylation of several carbohydrates. Unfortunately, the use of this milder acetylation catalyst for diacetylating diol **II** at the inventor's laboratory, even in a 1% molar amount, has turned out to be unselective, the ratio of chiral ketone **I** to elimination product IX being close to 62.5:37.5 (see example). Therefore, and due to the catalytic importance of chiral ketone **I,** it is necessary to find a preparation process for chiral ketone **I** which overcomes the deficiencies of the prior art.

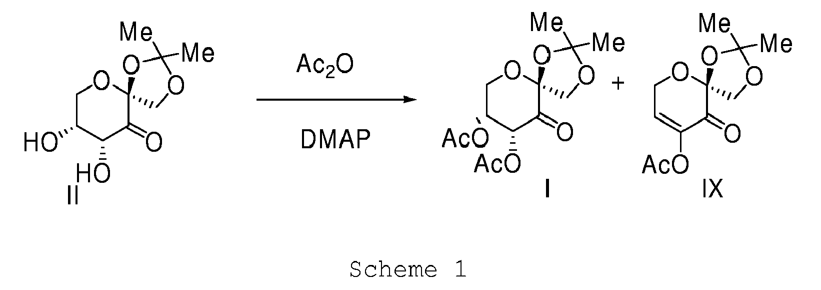

It is an object of the present invention to provide an efficient and scalable preparation process for chiral ketone I and for its enantiomeric counterpart in multi-gram amounts.

### Summary of the invention

It is an object of the present invention to provide a method for the preparation of chiral ketone I which overcomes the deficiencies of the prior art and which is easy to perform and scalable.

This object is achieved by the preparation process of the present invention.

The process for the preparation of chiral ketone I according to the present invention comprises the acetylation of diol II with an acetylating agent, characterized in that said acetylation is performed in the presence of a Lewis acid. More specifically, said acetylation is a diacetylation.

Surprisingly, the preparation process according to the present invention allows the preparation of chiral ketone I without substantial contamination by the elimination by-product IX. More specifically, the preparation process according to the present invention yields chiral ketone I in a ratio chiral ketone I to elimination product IX of at least 90:1, preferably at least 97:1, most preferably 99:1. In a preferred embodiment of the present invention, no substantial elimination product IX is detected by ¹H-NMR or chromatography in the reaction crude obtained through the preparation process of the present invention.

The term "acetylating agent" as defined in the invention refers to a compound capable of providing an acetyl radical through a nucleophilic attack (need of acetylating catalyst). Preferably, said acetylating agent is an acetic acid derivative, more preferably acetic anhydride. According to the preparation process of the present invention, the ratio between the acetylating agent and diol II is from 10:1 to 2:1, preferably 4:1.

In a preferred embodiment, the preparation process of ketone I according to the present invention further comprises a purification step, preferably a chromatographic filtration.

Advantageously, the preparation process of the present invention allows the preparation of chiral ketone I with high yield and selectivity. Moreover, the preparation process according to the present invention can be performed in multi-gram scale.

An advantage of the preparation process according to the present invention is that it is performed in mild conditions. Suitable temperatures for the performance of the acetylation reaction according to the present invention range from -10°C to 60°C. In a preferred embodiment of the present invention, said acetylation is performed at room temperature. In a further preferred embodiment of the present invention, said acetylation is performed in a very short period of time under thermal conditions (50°C). In another preferred embodiment of the present invention, said acetylation is performed under microwave irradiation.

A further advantage of the preparation process according to the present invention is that a mild acetylating catalyst is used, more specifically, a Lewis acid. Moreover, only a catalytic amount of Lewis acid is needed. Preferably, said Lewis acid is a group-II metal halide or a group-12 metal halide, most preferably selected between the group comprising zinc halide, magnesium halide and cadmium halide, for example zinc chloride.

In a preferred embodiment, the preparation process of ketone I according to the present invention comprises the following steps:
a) selective ketal cleavage on the substantially enantiomerically pure compound III, yielding the substantially enantiomerically pure diol II (see scheme 2);
b) acetylation of diol II with an acetylating agent, wherein said acetylation is performed in the presence of a Lewis acid.

Said selective ketal cleavage can be performed by known procedures, for example the procedure described by Shi and co-workers using substoichiometric amounts of DDQ in hydroorganic media followed by a purification step (Wu, X.-Y.; She, X.; Shi, Y. J. Am. Chem. Soc. 2002, 124, 8792-8793). Alternatively, in a further preferred embodiment of the present invention, said selective ketal cleavage can be performed by reacting compound III with a mixture of acid and water at room temperature, for example by the procedure described by Morris (Morris, P. E., Jr.; Kiely, D. E. J. Org. Chem. 1987, 52, 1149-52).

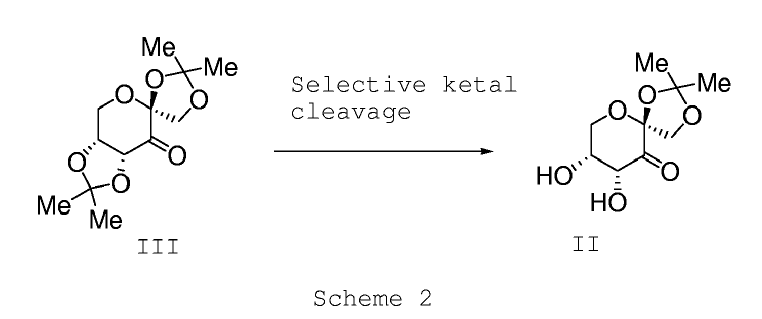

In a preferred embodiment, the preparation process of ketone I according to the present invention comprises the following steps:
a) oxidation of the substantially enantiomerically pure compound IV yielding the substantially enantiomerically pure compound III (see scheme 3);
b) selective ketal cleavage on the substantially enantiomerically pure compound III, yielding the substantially enantiomerically pure diol II (see scheme 2);
c) acetylation of diol II with an acetylating agent, wherein said acetylation is performed in the presence of a Lewis acid.

Said oxidation can be performed by known procedures. In a further preferred embodiment of the present invention, said oxidation is promoted by ruthenium, for example by the procedure reported by Mio (Mio, S.; Kumagawa, Y.; Sugai, S. Tetrahedron 1991, 47, 2133-44). Mio has described the quantitative oxidation of compound III to compound II by using catalytic amounts of a ruthenium precursor and sodium metaperiodate as the stoichiometric oxidant.

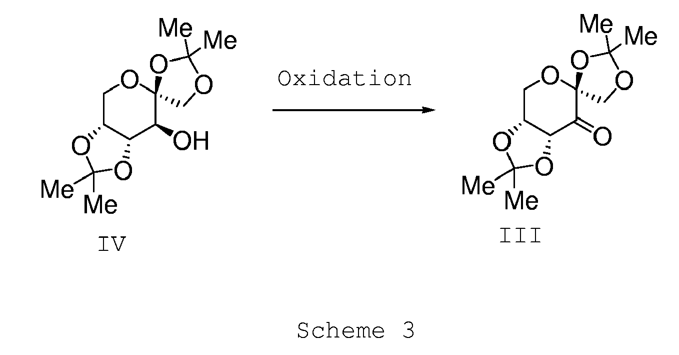

In a preferred embodiment, the preparation process of ketone I according to the present invention comprises the following steps:
a) reaction of D-fructose with acetone in the presence of acid, yielding the substantially enantiomerically pure compound IV (see scheme 4);
b) oxidation of the substantially enantiomerically pure compound IV yielding the substantially enantiomerically pure compound III (see scheme 3);
c) selective ketal cleavage on the substantially enantiomerically pure compound III, yielding the substantially enantiomerically pure diol II (see scheme 2);
d) acetylation of diol II with an acetylating agent, wherein said acetylation is performed in the presence of a Lewis acid.

According to this preferred embodiment, chiral ketone I is synthesized in four steps from D-fructose. Advantageously, D-fructose is a commercially available inexpensive product. The transformation of D-fructose to compound IV can be carried out through known procedures, for example by the procedure described by Kang and co-workers (Kang, J.; Lim, G. J.; Yoon, S. K.; Kim, M. Y. J. Org. Chem. 1995, 60, 564-577). Kang's procedure was made in acetone as both reagent and solvent at room temperature using sulphuric acid as the catalyst, and the product was purified through recrystallization.

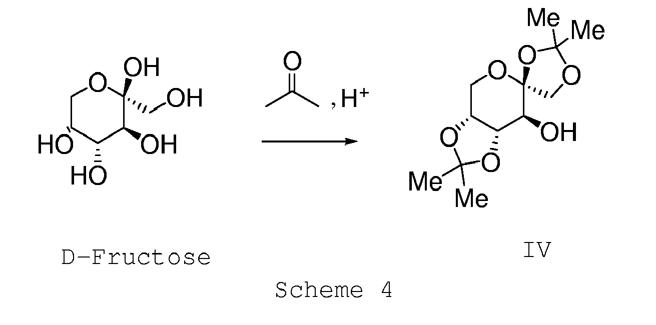

It is a further object of the present invention to provide a preparation process for chiral ketone V, the enantiomer of chiral ketone I. Chiral ketone V shares the features and advantages of chiral ketone I with regard to the epoxidation of alkenes and is the enantiomeric counterpart of chiral I, giving rise to epoxides whose optical activity is the opposite of the epoxides obtained in the same reaction conditions using chiral ketone I as catalyst. A person skilled in the art will understand that the reactivity features of chiral ketone **I** apply to chiral ketone **V,** the only difference between said compounds being their opposite stereochemistry and optical activity. The reactions catalysed by chiral ketone **I** will give rise to products with the opposite optical activity of those products obtained using chiral ketone **V** as catalyst instead of ketone **I,** in the same reaction conditions.

The process for the preparation of chiral ketone V according to the present invention comprises the acetylation of diol VI with an acetylating agent, characterized in that said acetylation is performed in the presence of a Lewis acid. More specifically, said acetylation is a diacetylation.

Advantageously, the preparation process according to the present invention allows the preparation of chiral ketone V without substantial contamination by the elimination by-product X (see scheme 5).

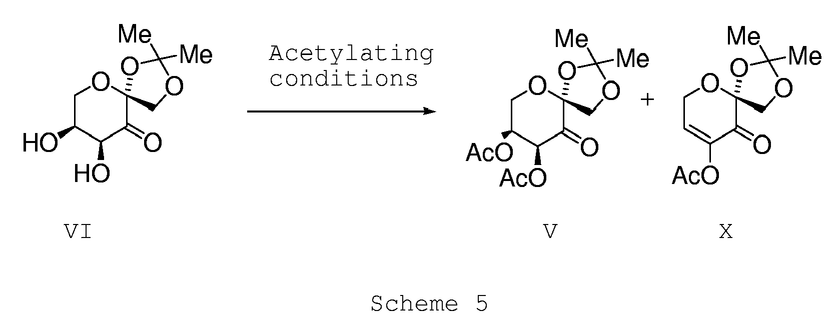

More specifically, the preparation process according to the present invention yields chiral ketone V in a ratio chiral ketone V to elimination product X of at least 90:1, preferably al least 97:1, most preferably 99:1. In a preferred embodiment of the present invention, no substantial elimination product X is detected by ¹H-NMR or chromatography in the reaction crude obtained through the preparation process according to the present invention.

Preferably, the acetylating agent is an acetic acid derivative, more preferably acetic anhydride. According to the preparation process of the present invention, the ratio between the acetylating agent and diol VI is from 10:1 to 2:1, preferably 4:1.

In a preferred embodiment, the preparation process of ketone V according to the present invention further comprises a purification step, preferably a chromatographic filtration.

Advantageously, the preparation process of the present invention allows the preparation of chiral ketone V with high yield and selectivity.

An advantage of the preparation process according to the present invention is that it is performed in mild conditions. The acetylation reaction according to the present invention can be carried out at a temperature from -10°C to 60°C. In a preferred embodiment of the present invention, said acetylation is performed at room temperature. In another preferred embodiment of the present invention, said acetylation is performed in a very short period of time under thermal conditions (50°C). In a further preferred embodiment of the present invention, said acetylation is performed under microwave irradiation.

A further advantage of the preparation process according to the present invention is that a mild acetylating catalyst is used, more specifically, a Lewis acid. Moreover, only a catalytic amount of Lewis acid is needed. Preferably, said Lewis acid is a group-II metal halide or a group-12 metal halide, most preferably selected between the group comprising zinc halide, magnesium halide and cadmium halide, for example zinc chloride.

In a preferred embodiment, the preparation process of ketone V according to the present invention comprises the following steps:
a) selective ketal cleavage on the substantially enantiomerically pure compound VII, yielding the substantially enantiomerically pure diol VI (see scheme 6);
b) acetylation of diol VI with an acetylating agent, wherein said acetylation is performed in the presence of a Lewis acid.

Said selective ketal cleavage can be performed by known procedures, for example the procedure described by Shi and co-workers using substoichiometric amounts of DDQ in hydroorganic media followed by a purification step (Wu, X.-Y.; She, X.; Shi, Y. J. Am. Chem. Soc. 2002, 124, 8792-8793). Alternatively, in a further preferred embodiment of the present invention, said selective ketal cleavage can be performed by reacting compound VII with a mixture of acid and water at room temperature, for example by the procedure described by Morris (Morris, P. E., Jr.; Kiely, D. E. J. Org. Chem. 1987, 52, 1149-52).

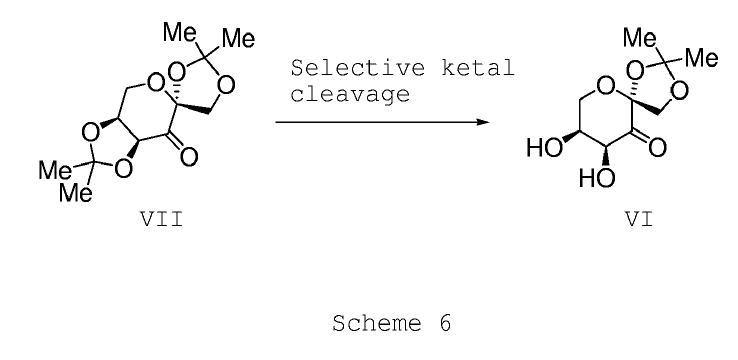

In a preferred embodiment, the preparation process of ketone V according to the present invention comprises the following steps:
a) oxidation of the substantially enantiomerically pure compound VIII yielding the substantially enantiomerically pure compound VII (see scheme 7);
b) selective ketal cleavage on the substantially enantiomerically pure compound VII, yielding the substantially enantiomerically pure diol VI (see scheme 6);
c) acetylation of diol VI with an acetylating agent, wherein said acetylation is performed in the presence of a Lewis acid.

Said oxidation can be performed by known procedures. In a further preferred embodiment of the present invention, said oxidation is promoted by ruthenium, for example by the procedure reported by Mio (Mio, S.; Kumagawa, Y.; Sugai, S. Tetrahedron 1991, 47, 2133-44). Mio has described the quantitative oxidation of compound III to compound II by using catalytic amounts of a ruthenium precursor and sodium metaperiodate as the stoichiometric oxidant.

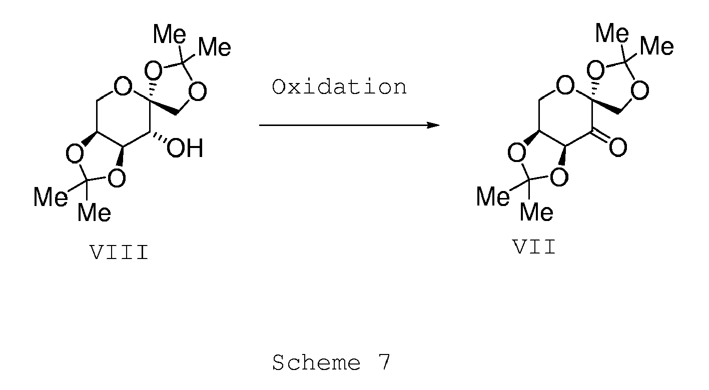

Compound VIII can be synthesised through known methods to a person skilled in the art. For example without being a limitation to the scope of the present invention, compound VIII can be synthesised from L-fructose, which in turn can be synthesised, with no limitation to the scope of the present invention, from L-sorbose. Alternatively, without being a limitation to the scope of the present invention, compound VIII can be synthesized from D-arabinose.

In a preferred embodiment, the preparation process of ketone V according to the present invention comprises the following steps:
a) reaction of L-fructose with acetone in the presence of acid (see scheme 8), yielding the substantially enantiomerically pure compound VIII;
b) oxidation of the substantially enantiomerically pure compound VIII yielding the substantially enantiomerically pure compound VII (see scheme 7);
c) selective ketal cleavage on the substantially enantiomerically pure compound VII, yielding the substantially enantiomerically pure diol VI (see scheme 6);
d) acetylation of diol VI with an acetylating agent, wherein said acetylation is performed in the presence of a Lewis acid.

The transformation of L-fructose to compound VIII can be made for example in acetone as both reagent and solvent at room temperature using sulphuric acid as the catalyst.

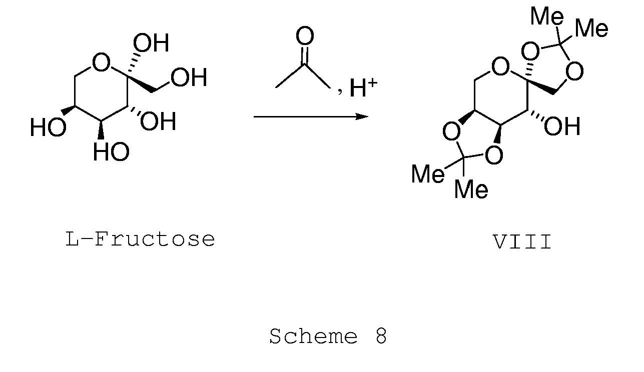

According to said preferred embodiment, chiral ketone V is synthesized in four steps from L-fructose. Advantageously, L-fructose can be synthesised by methods known to a person skilled in the art, for example, with no limitation to the scope of the present invention, from L-sorbose (see Wang, Z.-X.; Tu, Y.; Frohn, M.; Zhang, J.-R.; Shi, Y., J. Am. Chem. Soc., 1997, 119, 11224-11235; see also Chyi-Cheng Chen and Roy L. Whistler, Carbohydr. Res., 1988, 175, 265-271).

In a further preferred embodiment, the preparation process of ketone V according to the present invention comprises the following steps:
a) transformation of L-sorbose to L-fructose;
b) reaction of L-fructose with acetone in the presence of acid (see scheme 8), yielding the substantially enantiomerically pure compound VIII;
c) oxidation of the substantially enantiomerically pure compound VIII yielding the substantially enantiomerically pure compound VII (see scheme 7);
d) selective ketal cleavage on the substantially enantiomerically pure compound VII, yielding the substantially enantiomerically pure diol VI (see scheme 6);
e) acetylation of diol VI with an acetylating agent, wherein said acetylation is performed in the presence of a Lewis acid.

In another preferred embodiment, the preparation process of ketone V according to the present invention comprises the following steps:
a) transformation of D-arabinose to compound VIII;
b) oxidation of the substantially enantiomerically pure compound VIII yielding the substantially enantiomerically pure compound VII (see scheme 7);
c) selective ketal cleavage on the substantially enantiomerically pure compound VII, yielding the substantially enantiomerically pure diol VI (see scheme 6);
d) acetylation of diol VI with an acetylating agent, wherein said acetylation is performed in the presence of a Lewis acid.

Said transformation of D-arabinose to compound VIII can be performed according to methods known to a person skilled in the art (see for example Bessieres, B.; Morin, C., J. Org. Chem., 2003, 68, 4100-4103; see also Chyi-Cheng Chen and Roy L. Whistler, Carbohydr. Res., 1976, 52, 95-101).

In summary, the preparation process of ketone I, or alternatively ketone V, according to the present invention can be performed straightforward and in mild conditions, yielding substantially enantiomerically pure ketone I, or alternatively substantially enantiomerically pure ketone V, with high yield, chemoselectivity and stereoselectivity and in particular without substantial contamination by the corresponding elimination product, IX or X, respectively.

The present invention further relates to a method for producing an enantiomerically enriched epoxide from an alkene, said method comprising admixing said alkene, the enantiomerically enriched chiral compound I and an oxidizing agent, wherein said alkene is a non-electron deficient alkene. Alternatively, the present invention further relates to a method for producing an enantiomerically enriched epoxide from an alkene, said method comprising admixing said alkene, the enantiomerically enriched chiral compound V and an oxidizing agent, wherein said alkene is a non-electron deficient alkene.

Surprisingly, chiral ketone I, and alternatively, chiral ketone V, catalyse effectively the asymmetric epoxidation of non-electron deficient alkenes as well as electron deficient alkenes. As described by Shi, chiral ketone I and alternatively, chiral ketone V, are efficient catalysts for the epoxidation of electron deficient alkenes. Said electron deficient alkenes are less reactive than non-electron deficient alkenes, and thus, most chiral ketones used as catalysts for the epoxidation of alkenes failed to catalyse the epoxidation of electron-deficient alkenes. Chiral ketone I, and alternatively, chiral ketone V, have shown to be far much more reactive than other described chiral ketones, as said chiral ketones are capable of effectively catalysing the epoxidation of electron deficient alkenes. There is no example in the background art related to the use of chiral ketone I, or alternatively, chiral ketone V, for the epoxidation of non electron deficient alkenes. As high reactivity is often associated with low selectivity, a person skilled in the art would expect low selectivity in the epoxidation of non electron deficient alkenes with chiral ketone I, or alternatively, with chiral ketone V. Surprisingly, the inventors have found that the epoxidation of different alkenes using chiral ketone **I,** or alternatively, chiral ketone **V,** could be carried out straightforwardly.

Chiral ketone V shares the features and advantages of chiral ketone I with regard to the epoxidation of alkenes and is the enantiomeric counterpart of chiral ketone I, giving rise to epoxides whose optical activity is the opposite of the epoxides obtained in the same reaction conditions using chiral ketone I as catalyst. A person skilled in the art will understand that the reactivity features of chiral ketone **I** apply to chiral ketone **V,** the only difference between said compounds being their opposite stereochemistry and optical activity. The reactions catalysed by chiral ketone **I** will give rise to products with the opposite optical activity of those products obtained using chiral ketone **V** as catalyst instead of chiral ketone **I,** in the same reaction conditions.

In a preferred embodiment of the present invention, said non-electron deficient alkene is selected from the group comprising unfunctionalised alkenes, aryl substituted alkenes, hydroxyalkenes, *cis*-alkenes, trans-alkenes and trisubstituted alkenes.

The method for the asymmetric epoxidation of non-electron deficient alkenes according to the present invention can be performed in a variety of different sequences. For example, the oxidation agent can be added to a mixture comprising the chiral ketone and the alkene. Alternatively, the actual epoxidation agent (e.g. dioxirane) can be generated separately prior to contacting with the alkene.

In general, any oxidazing agent capable of providing the actual epoxidation agent (e.g. dioxirane) from chiral ketone I, or alternatively, from chiral ketone V, can be used in the present invention. A person skilled in the art will be able to select a suitable oxidizing agent from the group of oxidizing agents described in the background art. However, for economic reasons, a relatively inexpensive oxidizing agent is preferred, such oxidizing agent being selected from the group comprising oxone, peracids, hydrogen peroxide, sodium hypochlorite, potassium peroxomonosulfate, sodium perborate and hypofluoride.

In a preferred embodiment of the method of the present invention, the molar ratio of said chiral ketone I, or alternatively, said chiral ketone V relative to the amount of said alkene is not higher than 1 equivalent, preferably not higher than 0.3 equivalents, more preferably not higher than 0.1 equivalents.

Typically, any relatively inert solvent can be used in the method of the present invention. In a preferred embodiment, said solvent is a mixture aqueous-organic media. Typically, any relatively inert organic solvent can be used for the present invention, for example acetonitrile-dimethoxymethane.

In a preferred embodiment, the method of the present invention further comprises the addition of a phase-transfer catalyst. Said phase-transfer catalyst can be selected from the group comprising cationic nitrogen containing compounds and cationic phosphorous containing compounds, for example Bu₄NHSO₄.

In a further preferred embodiment, the method of the present invention can further comprise adjusting the pH from pH 6 to pH 8. The pH of the reaction solution can be conveniently achieved by adding sufficient amount of base or buffer to maintain the pH at the desired level. The base or buffer can be added separately, it can be added to the solution containing the compound, or it can be added to the solution containing the oxidizing agent. Any suitable bases and buffers known to a person skilled in the art can be used in the epoxidation method according to the present invention. Without being limited to, a particularly preferred buffer is based on potassium dihydrogen phosphate/potassium hydroxide.

The method of the present invention can be performed at a temperature ranging from -20°C to room temperature, preferably from -10°C to 0°C.

Advantageously, the epoxidation method according to the present invention provides epoxides with at least about 50% yield, more preferably at least about 60% yield and most preferably at least about 70% yield.

Advantageously, the epoxidation method according to the present invention provides epoxides with at least about 70% enantiomeric excess, more preferably about 80% enantiomeric excess and most preferably at least 90% enantiomeric excess.

The findings of the present invention show that chiral ketone **I** and chiral ketone **V,** respectively, are superior to other ketone based epoxidation catalysts described in the background art, as far as the alkene scope is concerned. More specifically, chiral ketone **I** and chiral ketone **V** can successfully catalyse the epoxidation of either electron deficient or non-electron deficient alkenes.

A further advantage of chiral ketone **I** and chiral ketone **V** as epoxidation catalysts is related to the catalyst robustness. In particular, chiral ketone **I** and chiral ketone **V** are not so easily degraded in the reaction media by an undesired Baeyer-Villiger reaction as other chiral ketone-based catalysts described in the background art.

A further advantage of chiral ketone **I** and chiral ketone **V** with regard to other chiral ketones used in the epoxidation of alkenes is that chiral ketone **I,** and alternatively chiral ketone **V** can be easily synthesized in mild conditions through the preparation process of the present invention.

Additional objects, advantages and features of the invention will become apparent to those skilled in the art upon examination of the description or may be learned by practice of the invention. The following examples are provided by way of illustration and are not intended to limit the scope of the invention. It is intended that the scope of the present invention be defined by the claims appended hereto.

### Detailed description of particular examples

### Examples of preparation of ketone I from compound II

In the following table some examples of preparation of chiral ketone I from compound II according to the process of the present invention (entries 4 to 7) are summarized and compared with other preparation processes of chiral ketone I described in the background art (entries 1 to 3).

**Table 1. Acetylation of compound II**

| | | | | |
|---|---|---|---|---|
| 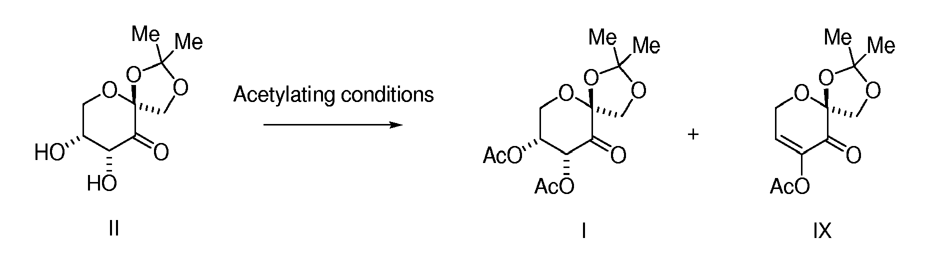 | | | | |

| Entry | Acetylating agent | Catalyst | Reaction conditions | Ratio I:IX^{a} |
|---|---|---|---|---|
| 1 | Ac₂O (3-fold molar excess) | DMAP (2 mol%) | DCM, rt, 16h | 64.4: 35.6^{b} |
| 2 | Ac₂O (3-fold molar excess) | DMAP (20 mol%) | DCM, rt, 16h | 1:99 |
| 3 | Ac₂O (8.7-fold molar excess) | NaOAc (1 mol%) | Ac₂O; 50°C; 5 min, 50°C | 62.5: 37.5 |
| 4 | Ac₂O (8.7-fold molar excess) | ZnCl₂ (1 mol %) | Ac₂O; 50°C; 5 min, 50W^{c} | 99:1 |
| 5 | Ac₂O (8.7-fold molar excess) | ZnCl₂ (1 mol %) | Ac₂O; 50°C; 15 min | 99:1 |
| 6 | Ac₂O (6-fold molar excess) | ZnCl₂ (1 mol %) | Ac₂O; rt; 3h | 99:1 |
| 7 | Ac₂O (4-fold molar excess) | ZnCl₂ (2.5 mol %) | Ac₂O; rt; 3h | 99:1 |
| | | | | |

| | | | | |
|---|---|---|---|---|
| ^{a} Measured by ¹H-NMR after performing the same work-up as described in Wu, X.-Y.; She, X.; Shi, Y. J. Am. Chem. Soc. 2002, 124, 8792-8793. ^{b} This value refers to the diacetylation of diol II as the raw material from the selective deketalisation step. A 74.6:25.4 ratio was observed when chromatographically pure starting material II was used. ^{c} Reactions carried out in a CEM Discover Microwave Reactor. | | | | |

Entries 1 and 2 correspond to the process for the preparation of chiral ketone I according to Shi and coworkers. These results show that the preparation process described by Shi leads to variable amounts of the elimination product IX being formed together with the desired compound I.

On the other hand, the use of sodium acetate under microwave irradiation has been reported to be a useful catalyst for the acetylation of several carbohydrates. Disadvantageously, the use of this milder acetylation catalyst for diacetylating diol **II** at the inventor's laboratory, even in a 1% molar amount, has turned out to be unselective, the ratio of chiral ketone **I** to elimination product **IX** being close to 62.5:37.5 (entry 3).

Advantageously, in contrast to the processes for the preparation of ketone I described in the background art, the process according to the present invention leads to compound I in high yield and purity, as table 1 shows.

Therefore, the preparation process of chiral ketone I, or alternatively, chiral ketone V, is superior to the preparation processes described in the prior art, as it is characterized by mild reaction conditions, easy work-up procedures and high reaction yields and selectivities.

### Examples of epoxidation of non-electron deficient alkenes catalysed by ketone I

In the following table the results of asymmetric epoxidation of a variety of non-electron deficient alkenes using chiral ketone I as a catalyst and oxone as oxidating agent are summarised.

**Table 2. Epoxidation examples using chiral ketone I**

| | | | | | |
|---|---|---|---|---|---|
| 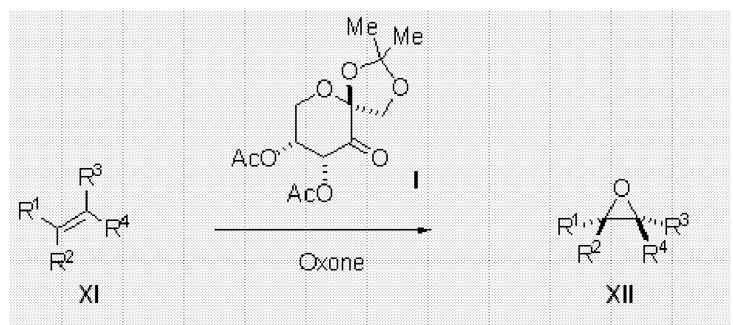 | | | | | |

| Entry | Alkene | Reaction conditions | Yield | Ee (conf.) | Ee enrichment^{a} |
|---|---|---|---|---|---|
| 1 | 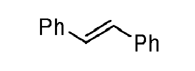 | 10 mol% I, 0°C, 2^{b}+16^{c}h | 53% ^{d} | 90% ^{d}, (*R*,*R*)-XIIa | Hexane, 46%, >99% ee |
| 2 | 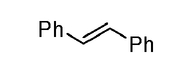 | 30 mol% I, 0°C, 2^{b}+16^{c}h | 75% | 94%, (*R,R*)-XIIa | -- |
| 3 | 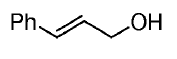 | 10 mol% I, 0°C, 2^{b}+16^{c}h | 68% | 96%, (*R,R*)-XIIb | -- |
| 4 | 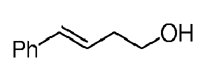 | 8 mol% I, 0°C, 2^{b}+16^{c}h | 67% | 81%^{e}, (*R*)-XIIc | -- |
| 5 | 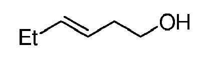 | 30 mol% I, 0°C, 2^{b}+16^{c}h | ^{f} | 83%^{e}, (*R,R*)-XIId | -- |
| 6 | 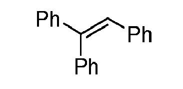 | 8 mol% I, 0°C, 2 ^{b}+16^{c}h | 58% | 87%, (*R*)-XIIe | Hexane, 45%, 90% ee |

| | | | | | |
|---|---|---|---|---|---|
| ^{a} The epoxide was recrystallized after chromatography in order to improve its ee. The solvent, overall yield and ee are shown. ^{b} Oxone and base addition times. ^{c} Reaction time. ^{d} Mean value of 4 experiments. ^{e} In this case 7.2 eq. of K₂CO₃ were added. The pH-value increased from 6 to 10. ^{f} Not determined (epoxyalcohol XIId is distilled together with the solvents during the work-up). | | | | | |

The reactions were carried out normally with a 10% molar amount of catalyst, which was increased up to 30% molar (standard catalyst amount for the standard Shi's catalyst **2**) in the difficult cases. The epoxidations were carried out normally at 0°C as this temperature offered a good balance between conversion and selectivity at that catalyst loading. However, the reaction mixture could be cooled up to -10°C in order to improve the selectivity.

Surprisingly, as shown in the Table above, chiral ketone I shows to be an excellent catalyst for the asymmetric epoxidation of non-electron deficient alkenes.

## Claims

1. A process for the preparation of chiral ketone I comprising the acetylation of diol II with an acetylating agent, wherein said acetylation is performed in the presence of a Lewis acid.

2. The process of claim, wherein said acetylating agent is an acetic acid derivative.

3. The process according to any of claims 1 or 2, wherein said Lewis acid is a metal halide selected between the group comprising zinc halide, magnesium halide and cadmium halide.

4. The process according to any of claims 1 to 3, comprising the following steps:
a) selective ketal cleavage on the substantially enantiomerically pure compound III, yielding the substantially enantiomerically pure diol II (see scheme 2);
b) acetylation of diol II with an acetylating agent, wherein said acetylation is performed in the presence of a Lewis acid.

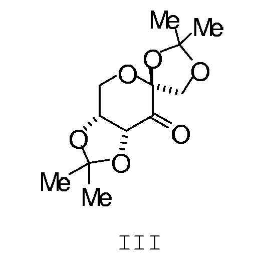

5. The process according to any of claims 1 to 3, comprising the following steps:
a) oxidation of the substantially enantiomerically pure compound IV yielding the substantially enantiomerically pure compound III (see scheme 3);
b) selective ketal cleavage on the substantially enantiomerically pure compound III, yielding the substantially enantiomerically pure diol II (see scheme 2);
c) acetylation of diol II with an acetylating agent, wherein said acetylation is performed in the presence of a Lewis acid.

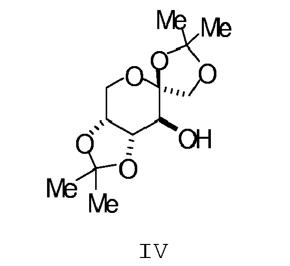

6. The process according to any of claims 1 to 3, comprising the following steps:
a) reaction of D-fructose with acetone in the presence of acid, yielding the substantially enantiomerically pure compound IV (see scheme 4);
b) oxidation of the substantially enantiomerically pure compound IV yielding the substantially enantiomerically pure compound III (see scheme 3);
c) selective ketal cleavage on the substantially enantiomerically pure compound III, yielding the substantially enantiomerically pure diol II (see scheme 2);
d) acetylation of diol II with an acetylating agent, wherein said acetylation is performed in the presence of a Lewis acid.

7. A process for the preparation of chiral ketone V, comprising the acetylation of diol VI with an acetylating agent, wherein said acetylation is performed in the presence of a Lewis acid.

8. The process of claim 7, wherein said acetylating agent is an acetic acid derivative.

9. The process according to any of claims 7 or 8, wherein said Lewis acid is a metal halide selected between the group comprising zinc halide, magnesium halide and cadmium halide.
